# EUROPEAN PATENT APPLICATION

(11) **EP 4 545 074 A1**
(43) Date of publication of application: **30.04.2025**
(21) Application number: 23206367.7
(22) Date of filing: 27.10.2023
(51) Int. Cl.: A61K 31/00, A61K 9/20

(54) **PHARMACEUTICAL COMPOSITION OF FURAZIDIN FOR VAGINAL ADMINISTRATION**

(71) Applicant: Adamed Pharma S.A., 05-152 Czosnow (PL)
(72) Inventor: KUZNIEWSKA, Magdalena, 01-887 Warszawa (PL); NIEMCZYK, Katarzyna, 32-600 Oswiecim (PL); KIRYLUK, Anna, 05-800 Pruszkow (PL); WENCLAW-WALESIAK, Marta, 02-989 Warszawa (PL)
(74) Representative: Sulikowski, Daniel

(57) **Abstract**

A pharmaceutical composition of furazidin in the form of a vaginal mucoadhesive tablet, said tablet comprising or consisting of: a) granulate containing furazidin, a pharmaceutically acceptable polymeric mucoadhesive binder, and intragranular pharmaceutically acceptable excipients, said excipients comprising or consisting of one or more intragranular fillers and an intragranular disintegrant; and b) extragranular pharmaceutically acceptable excipients, said excipients comprising or consisting of one or more extragranular fillers, an extragranular disintegrant, and a lubricant, and wherein the tablet comprises from 1 to 15% by weight of furazidin with respect to the weight of the tablet.

## Description

### Field of the invention

The invention relates to a pharmaceutical composition comprising furazidin in the form of a tablet for vaginal administration and a process of its preparation. The composition can find use in the local treatment of bacterial vaginitis.

### Prior art

Furazidin (Furagin, 1-[3-(5-nitro-2-furyl)-2-propenylideneamino-imidazolidine-2,4-dione) is a medicament widely used in the oral treatment of acute and chronic bacterial urinary tract infections (UTI), prostate infections, recurrent urinary tract infections, after surgery of the urinary tract, and prostate inflammation. Pharmaceutical dosage forms for oral administration are tablets, liquid suspensions, and powders or granulates for preparing liquid suspensions. Various furazidin tablets designed for oral administration are present on the market.

WO2022157357 discloses prolonged-release pharmaceutical composition of furazidin for oral administration in the treatment of urinary tract infections (UTI). The prolonged-release pharmaceutical composition for oral administration comprises: a) an immediate release component, comprising furazidin and one or more pharmaceutically acceptable excipients; and b) a modified-release component, comprising furazidin, a controlled-release agent, and one or more pharmaceutically acceptable excipients. In exemplary embodiments tablets have high furazidin content, about 32% to 50% by weight in the immediate release component, and about 40 to 60% by weight in the modified-release component.

WO2018069804 discloses pharmaceutical composition in the form of a powder or granulate or coated granulate for oral administration in the treatment of urinary tract infections as a suspension or as a capsule filling, containing from 0.5% to 95% by weight of furazidin, from 5% to 99% by weight of a bulking agent, and 0.1% to 30% by weight of a binder. A granulate designed for the preparation of mini-tablets to be used as a capsule filling was also disclosed. The granulate contains about 64% by weight of furazidin, sucrose as a filler, a mixture of acrylate/methacrylate polymers as a binder, talc and magnesium stearate, and is prepared by wet granulation in a fluidized bed granulator.

It has been recently reported that furazidin can also be used in the local treatment of bacterial vaginitis. WO2018188992 discloses furazidin for vaginal use in the treatment of a bacterial vaginal infection, in particular infection caused by Gardnerella vaginalis and/or Atopobium vaginae bacteria. Semi-solid cream and cocoa butter-based globules are also disclosed and tested for local antibacterial activity. Also proposed is exemplary tablet form prepared by direct compression, comprising 69% or 44% by weight of furazidin, and further comprising lactose, silicon dioxide, and magnesium stearate.

It has been found by the present inventors that prior art vaginal tablet obtained by direct compression of the blend of furazidin with lactose, silicon dioxide and magnesium stearate as disclosed in WO2018188992 is not suitable for vaginal administration. The tablet does not enable proper residence time and bioavailability of furazidin at the site of administration.

Still, vaginal administration in the form of a vaginal tablet is advantageous in the local treatment of bacterial vaginitis over semi-solid forms for several reasons. The tablet is more convenient for application and insertion into vaginal cavity. Tablet is also advantageous over semi-solid intravaginal globules, mainly because globules soften and are prone to leakage at the body temperature, leading to low residence time, messiness and discomfort. Another issue to consider in the development of clinical dosage forms is that formulations that involve use of a plastic applicator could lead to problems with disposal of applicators.

Tablets offer further advantages, such as portability, precise dosing, high stability, ease of storage, handling and administration and feasibility of large-scale production at a low cost compared to semi-solid systems.

The aim of the invention was to provide furazidin in the form of a vaginal tablet that is easy applicable, without the use of any applicator-type device, so as to increase patient comfort and compliance.

The aim of the invention was also to provide furazidin in the form of a vaginal tablet that includes low percentage of furazidin and despite of this enables even distribution of the active substance. The aim of the invention was also to provide furazidin in the form of a vaginal tablet having prolonged residence time in the place of its application, i.e. vaginal cavity, and therefore increase of bioavailability and reduction of required dosage frequency. This is important in the case of furazidin, a substance having very low water solubility, the more important when vaginal administration is concerned when very low water amount is available at the site of administration for disintegration and dissolution.

Furthermore, to enable easy manipulation and insertion without any applicator, a vaginal tablet should have quite large dimensions. As a consequence, the percentage of furazidin in the tablet would be quite low, much lower than in tablets designed for oral administration. This leads to the problems with required content uniformity of tablets. The problem of uniformity in the case of tablets produced by direct compression should be dealt with by the use of a premix furazidin/silicon dioxide with relatively high amounts of silicon dioxide.

Therefore, there is a need for a furazidin vaginal tablet that enables even distribution, high residence time and fast disintegration in the site of administration. There is also a need for furazidin vaginal tablet of high dimensions and low furazidin content without loss of content uniformity, i.e. ensuring at the same time suitable average content and uniformity of furazidin content throughout the batch of the tablets during tableting. Furthermore, there is a need for a tablet that can be prepared in a smooth industrial process, from easy processable tableting mass.

### Summary of the invention

According to the invention, there is provided a pharmaceutical composition of furazidin for vaginal administration in the form of a tablet that fulfills above mentioned criteria, as well as a process of preparation of such a composition.

According to the invention, a pharmaceutical composition of furazidin is in the form of a vaginal mucoadhesive tablet, comprising or consisting of:
a) a granulate containing furazidin, a pharmaceutically acceptable mucoadhesive polymeric binder and intragranular pharmaceutically acceptable excipients, said excipients comprising or consisting of one or more intragranular fillers and an intragranular disintegrant; and
b) extragranular pharmaceutically acceptable excipients, said excipients comprising or consisting of one or more extragranular fillers, an extragranular disintegrant and a lubricant,
and wherein the tablet comprises from 1 to 15% by weight of furazidin with respect to the tablet.

According to the invention there is also provided a process of the preparation of a pharmaceutical composition of furazidin in the form of a mucoadhesive tablet for vaginal administration, said process comprising or consisting of the following steps:
a) granulation of a mixture of furazidin and intragranular excipients with an aqueous solution of a mucoadhesive binder to prepare a granulate;
b) adding extragranular excipients to the granulate to prepare a blend, and
c) compressing the blend into the tablet.
The tablet of the invention or prepared according to the process of the invention is useful in treating bacterial vaginitis by local administration, i.e. by administration into vaginal cavity.

### Detailed description of the invention

The term "vaginal tablet" relates to the tablet that is destined for administration to the vaginal cavity, but otherwise is prepared from the pharmaceutically acceptable excipients that are conventionally used for the preparation of conventional oral tablets and using conventional tableting techniques known as such in the field of pharmaceutical technology. It has therefore advantage of easy manufacture using conventional tableting process.

Vaginal tablet comprises furazidin in a matrix comprising mucoadhesive polymer (i.e. polymeric binder) that is hydrophilic and thus wettable/swellable and able to form a gel in water environment.

The term "mucoadhesive" refers to the phenomenon of mucoadhesion, i.e. the state in which due to the presence of a mucoadhesive polymer (i.e. polymeric binder) the bond, such as a physical one, is formed for a period of time between vaginal mucosa and the tablet by means of interfacial forces.

The result of the mucoadhesion is the adherence of the tablet to the vaginal mucosa in the intimate contact due to wetting and swelling to disintegrate in the vagina and release the drug in a controlled manner by diffusion through swollen mucoadhesive polymer and progressive dissolution of the gel matrix.

The terms "granulate", "intragranular", "extragranular", "excipients", "binder", "filler", "disintegrant", "lubricant" have the conventional meaning such as commonly used in the technology of oral solid forms.

By excipient it is meant any component other than the active ingredient furazidin, i.e. pharmaceutically acceptable inactive ingredient that is commonly used to prepare tablets for oral administration.

Intragranular excipients are the fraction of excipients added during the granulation process and present in the granulate obtained.

Extragranular excipients are the fraction of excipients added as a powder to the granulate after granulation process to obtained a blend and before compression or tableting process of the blend.

The mucoadhesive tablet of the invention comprises 1 to 15% by weight of furazidin.

Preferably, the tablet of the invention comprises 2 to 10%, such as 2.5 to 10% by weight of furazidin.

In one embodiment, the tablet of the invention comprises 2.5% by weight of furazidin.

In another embodiment, the tablet of the invention comprises 10% by weight of furazidin.

The tablet of the invention may comprise from 2.5 to 100 mg of furazidin per unit dosage form, i.e. tablet (furazidin dose strength). In specific embodiments, furazidin dose strength is selected from the group consisting of 5 mg, 25 mg, 50 mg and 100 mg. Preferably, tablet of the invention comprises 5 mg of furazidin per unit dosage form.

In one embodiment, the tablet of the invention comprises 2.5% by weight of furazidin and furazidin dose strength is 5 mg.

In another embodiment, the tablet of the invention comprises 10% by weight of furazidin and furazidin dose strength is selected from the group consisting of 25 mg, 50 mg and 100 mg.

In a specific embodiment, the tablet of the invention comprises 10% by weight of furazidin and furazidin dose strength is 25 mg.

In another specific embodiment, the tablet of the invention comprises 10% by weight of furazidin and furazidin dose strength is 50 mg.

In yet another specific embodiment, the tablet of the invention comprises 10% by weight of furazidin and furazidin dose strength is 100 mg.

The tablet of the invention comprises granulate a), wherein furazidin is included in a matrix comprising mucoadhesive polymeric binder.

The amount of the polymeric mucoadhesive binder in the tablet of the invention is in the range from 2 to 7%, especially 3% by weight with respect to the weight of the tablet.

The mucoadhesive polymeric binder may be selected from the group consisting of povidone, hydroxypropylmethylcelluloses (HPMC), such as HPMC-E15, hydroxypropylcelluloses (HPC), such as Klucel MXF, high molecular weight acrylic acid polymer crosslinked with divinyl glycol (polycarbophil), such as Noveon AA-1, carboxypolymethylenes and carbomers, such as Carbopol 974 NF, and alginates, such as Protanal PH 6160.

Preferred mucoadhesive polymeric binder is povidone, especially povidone K30.

Povidone, as commonly recognized in the field of pharmaceutical technology, means polyvinylpyrrolidone.

Granular and extragranular excipients comprise one or more intragranular and extragranular fillers, respectively.

The amount of one or more intragranular fillers is in the range from 20 to 80% by weight with respect to the weight of the tablet.

The amount of one or more extragranular fillers is in the range from 20 to 40% by weight with respect to the weight of the tablet.

The total amount (sum) of intragranular and extragranular fillers in the entire tablet is in the range from 1 to 97.5% by weight with respect to the weight of the tablet.

The intragranular and extragranular fillers may be water soluble or water insoluble one.

The presence of water soluble and insoluble fillers and their respective weight ratio may allow to modify release properties of the tablet.

Advantageously, the intragranular excipients comprise a mixture of water soluble and water insoluble intragranular fillers.

Also advantageously, the extragranular excipients comprise a mixture of water soluble and water insoluble extragranular fillers.

In a particular embodiment, the intragranular excipients comprise a mixture of water soluble and water insoluble intragranular fillers and the extragranular excipients comprise a mixture of water soluble and water insoluble extragranular fillers.

In particular, the weight ratio of water soluble filler to water insoluble filler in the tablet is in the range from 3.4 : 5.6 to 7.6 : 11.9.

Water insoluble intragranular and/or extragranular filler may be selected from the group consisting of microcrystalline cellulose, calcium phosphate tribasic, dibasic calcium phosphate, calcium sulphate and dicalcium phosphate. Preferred water insoluble filler is microcrystalline cellulose.

Water soluble intragranular and/or extragranular filler may be selected from the group consisting of lactose, sorbitol, xylitol, mannitol, amylose, dextrose and soluble hydroxyalkylcelluloses. Preferred water soluble filler is lactose, especially lactose monohydrate.

Especially preferred said one or more intragranular and/or extragranular filler is a mixture of microcrystalline cellulose and lactose, especially lactose monohydrate.

Advantageously, the weight ratio of lactose monohydrate to microcrystalline cellulose is 2.8 : 5.3.

In a preferred embodiment, intragranular excipients fillers and extragranular fillers are the same.

Accordingly, especially preferred said one or more intragranular fillers is a mixture of microcrystalline cellulose and lactose, especially lactose monohydrate, and said one or more extragranular fillers is a mixture of microcrystalline cellulose and lactose, especially lactose monohydrate.

In a specific embodiment, said one or more intragranular fillers is a mixture of microcrystalline cellulose and lactose, especially lactose monohydrate, said one or more extragranular fillers is a mixture of microcrystalline cellulose and lactose, especially lactose monohydrate, and mucoadhesive polymeric binder is povidone, especially povidone K30.

Intragranular and extragranular excipients comprise an intragranular and extragranular disintegrant, respectively.

Intragranular and extragranular disintegrant may be selected from disintegrants conventionally used in tablets.

Intragranular and extragranular disintegrant may be in particular selected from the group consisting of starch sodium glycolate, corn starch pregelatinised, such as corn starch pregelatinised 1500, low-substituted hydroxypropylcellulose, cellulose microcrystalline, croscarmellose sodium and crospovidone.

Preferred intragranular and extragranular disintegrant is crospovidone, i.e. crosslinked polyvinylpyrrolidone.

Preferably, the intragranular and extragranular disintegrant is the same and is crospovidone.

The total amount of disintegrant in the tablet is in the range from 2.0 to 7.0% by weight with respect to the weight of the tablet. The weight ratio of the disintegrant in the granulate to the disintegrant in the extragranular excipients is 2:3.

Extragranular excipients comprise lubricant.

The amount of the lubricant is in the range from 0.5 to 2.0% by weight with respect to the weight of the tablet.

The lubricant may be selected from the group consisting of calcium stearate, stearic acid, palmitic acid, stearyl alcohol, polyethylene glycols (PEG), hydrogenated castor oil, hydrogenated plant oils, magnesium stearate and sodium stearyl fumarate.

Preferred lubricant is selected from the group consisting of magnesium stearate and sodium stearyl fumarate. Especially preferred is magnesium stearate.

The tablet of the invention preferably has the shape that is convenient for insertion into vagina without using any device, such as applicator. Advantageously, the tablet is almond-shaped, oval-shaped, bullet-shaped or capsule-shaped. Preferred is almond-shaped tablet pointed out at its one end.

According to the needs, if treatment of mixed vaginal infections is desired, the tablet may include further antimicrobial agent useful in the treatment of vaginal infections selected from the group consisting of antibacterial, antifungal and antiparasitic agents.

Tablet of the invention may be provided with a non-functional coating, such as colored aesthetic coating. A coating may a typical customary, ready for use coating, such as polyvinyl alcohol based coating, like Opadry.

Advantageous is the composition of the invention wherein the granulate is prepared by wet-granulation, especially by high-shear granulation.

It will be appreciated by a skilled person that wet-granulation is a granulation process, in particular using high-shear granulator, wherein a blend of furazidin with intragranular excipients is prepared and dry mixed, a granulating liquid, such as an aqueous solution of a binder is added to the blend, and wetted blend whole is granulated with a mixer and chopper.

The object of the invention is also a process of the preparation of a pharmaceutical composition of furazidin in the form of a mucoadhesive tablet for vaginal administration, comprising or consisting of the following steps:
a) granulation of the blend of furazidin and pharmaceutically acceptable intragranular excipients with an aqueous solution of a polymeric mucoadhesive binder to prepare furazidin granulate;
b) adding pharmaceutically acceptable extragranular excipients to the furazidin granulate to prepare a blend, and
c) compressing the blend into the tablet.

Preferably, granulation is performed in a high-shear granulator.

The tablet may be also coated in a conventional coating process.

Techniques for use in the process of the invention are as such commonly known by a skilled person.

### Example 1. Preparation of the tablets of the invention

Almond-shaped tablets of the furazidin strength 5 mg, 25 mg, 50 mg and 100 mg and total weight of the tablet 200 mg, 250 mg, 500 mg and 1000 mg, respectively were prepared. Composition of the tablets is presented in Table 1 below. Percentages of the granulate and extragranular phase are given in % by weight per whole tablet.

**Table 1. Composition of the tablets of the invention for furazidin strength 25, 50, 100 and 5 mg**

| | | 25 mg | | 50 mg | | 100 mg | | 5 mg | |
|---|---|---|---|---|---|---|---|---|---|
| Component | Function | mg | % * | mg | % * | mg | % * | mg | % * |
| Granulate | | | | | | | | | |
| Furazidin | Active substance | 25.00 | 10% | 50.00 | 10% | 100.00 | 10% | 5.00 | 2.5% |
| Lactose, monohydrate | Filler | 100.00 | 40% | 200.00 | 40% | 400.00 | 40% | 95.00 | 43.5% |
| Microcrystalline cellulose, | Filler | 37.50 | 15% | 75.00 | 15% | 150.00 | 15% | 30.00 | 19% |
| Crospovidone (Polyplasdone XL) | Disintegrant | 5.00 | 2% | 10.00 | 2% | 20.00 | 2% | 4.00 | 2% |
| Povidone K30 | Binder | 7.50 | 3% | 15.00 | 3% | 30.00 | 3% | 6.00 | 3% |

| Extragranular phase | | | | | | | | | |
|---|---|---|---|---|---|---|---|---|---|
| Lactose, monohydrate, | Filler | 32.50 | 13% | 65.00 | 13% | 130.00 | 13% | 26.00 | 13% |
| Microcrystalline cellulose, | Filler | 32.50 | 13% | 65.00 | 13% | 130.00 | 13% | 26.00 | 13% |
| Crospovidone (Polyplasdone XL) | Disintegrant | 7.50 | 3% | 15.00 | 3% | 30.00 | 3% | 6.00 | 3% |
| Magnesium stearate | Lubricant | 2.50 | 1% | 5.00 | 1% | 10.00 | 1% | 2.00 | 1% |

| Per whole tablet | | | | | | | | | |
|---|---|---|---|---|---|---|---|---|---|
| Furazidin | Active substance | 25.00 | 10% | 50.00 | 10% | 100.00 | 10% | 5.00 | 2.5% |
| Lactose, monohydrate | Filler | 132.50 | 53% | 265.00 | 53% | 530.00 | 53% | 121.00 | 56.5% |
| Microcrystalline cellulose | Filler | 70.00 | 28% | 140.00 | 28% | 280.00 | 28% | 56.00 | 22% |
| Crospovidone (Polyplasdone XL) | Disintegrant | 12.50 | 5% | 25.00 | 5% | 50.00 | 5% | 10.00 | 5% |
| Povidone K30 | Binder | 7.50 | 3% | 15.00 | 3% | 30.00 | 3% | 6.00 | 3% |
| Magnesium stearate | Lubricant | 2.50 | 1% | 5.00 | 1% | 10.00 | 1% | 2.00 | 1% |
| Total mass of the tablet | | 250 mg | | 500 mg | | 1000 mg | | 200 mg | |
| Dimension of the tablet in mm | | 7.80 × 12.50 | | 10.00 × 16.00 | | 18.00 × 11.00 or 19 × 12 or 20x12 | | 7.30x11.70 | |

| | | | | | | | | | |
|---|---|---|---|---|---|---|---|---|---|
| * % by weight per whole tablet | | | | | | | | | |

Tablets have been prepared using wet-granulation technique and a high-shear granulator apparatus.

An aqueous solution of povidone K30 was prepared as a granulation liquid at 20% by weight. Furazidin was blended with the part of lactose to prepare a furazidin premix. The furazidin premix was mixed in a mixing bowl of a high-shear granulator with microcrystalline cellulose and crospovidone to prepare a powder blend. The powder blend was fed with the granulation liquid and granulated using a high-speed impeller to obtain a wet mass. The wet mas was milled, forced through the sieve to produce wet granules, and dried. Dry granulate was mixed with extragranular components to obtain a tableting mass. Tableting mass was then compressed into tablets. The tablets were subsequently coated with a yellow Opadry polyvinyl alcohol based coating.

### Example 2. Comparative tablets

Comparative tablets having composition presented in Table 2 below, which is the same as the total composition of the tablets of Example 1 for furazidin strength 50 mg were prepared without granulation.

| Component | % by weight/tablet |
|---|---|
| Furazidin (50 mg) | 10% |
| Lactose, monohydrate | 53% |
| Microcrystalline cellulose | 28% |
| Crospovidone (Polyplasdone XL) | 5% |
| Povidone K30 | 3% |
| Magnesium stearate | 1% |

Premix of furazidin with magnesium stearate was prepared and then mixed with the rest of the components to obtain a homogenous blend. The blend was compressed using direct tableting technique, and tablets were coated with yellow polyvinyl alcohol based coating to produce comparative tablets.

### Example 3. Measurement of properties of tablets

### A. Mucoadhesion of the tablets

Tablets' capacity to adhere to the vaginal mucosa at the time of administration was assessed *in vitro* by measurement of the work necessary for its detachment from the mucosa (adhesion work) by means of the TA.XTplus Texture Analyser device and Exponent Connect software (both from Stable Micro Systems). The Mucoadhesion Rig, 36 mm diameter stainless-steel cylinder probe, 5 kg loadcell, and the "Measure Distance in Compression" mode with the following program of the test were used:
Pre-Test Speed: 0.5 mm/s
Test speed: 0.1 mm/s
Post-Test Speed: 0.1 mm/s
Target Mode: Force
Applied Force: 0.2 N
Contact time: 30 s
Trigger Type: Force

Mucin disc (10% mucin solution) was used as an *in vitro* mono-component mucosa model.

Mucin disc was prepared by evenly applying 10% mucin solution on a cellulose filter and sticking it to the underside of the probe with double-sided adhesive tape.

Vaginal Fluid Simulant (VFS), pH=4.5, was used to mimic the environment of vaginal mucosa.

VFS was prepared as described in "A Vaginal Fluid Simulant, Derek H. Owen* and David F. Katz, Contraception 1999;59:91-95, except that pH was adjusted to 4.5 instead of 4.2.

Accordingly, the formulation for 1 L of solution given as compound and weight (g), was as follows: calcium, 0.120; potassium, 0.978; sodium, 1.38; chloride, 2.13; albumin, 0.018; lactic acid, 2.00; acetic acid, 1.00; glycerol, 0.16; urea, 0.4; and glucose, 5.0. A specific "recipe" for 1 L of this simulant given as compound and weight (g), is as follows: NaCl, 3.51; KOH, 1.40; Ca(OH)2, 0.222; bovine serum albumin, 0.018; lactic acid, 2.00; acetic acid, 1.00; glycerol, 0.16; urea, 0.4; and glucose, 5.0. Once these compounds were combined, the mixture was adjusted to pH of 4.5 using HCl.

Mucoadhesion Rig was thermostated at 37°C. The samples were then prepared by fixing the whole tested tablet (n = 5 for each test) to the Mucoadhesion Rig with double-sided adhesive tape.

0.6 g of the Vaginal Fluid Simulant was applied onto the tablet fixed to the Mucoadhesion Rig. The probe with the mucin disc was moved downward at a pre-test speed 0.5 mm/s until it came into contact with the tablet, compressed at the test speed 0.1 mm/s to begin the bonding process, and predetermined compression force 0.2N force was applied and maintained for the dwell time 30 s.

The probe was then separated from the tablet at the speed of 0.1 mm/s until the complete detachment of the disc from the tablet. During the tests, force was measured and recorded as a function of displacement. From these adhesion curves the work of adhesion (the absolute area under the curve) and the force of detachment (the maximum of the force) are determined.

Mucoadhesion (as the work of adhesion) of the tablets of the invention (Ex. 1) prepared using wet-granulation technique and comparative tablets of the same composition prepared by direct compression without granulation (Ex. 2) are 0.74 and 0.41, respectively, as presented in Table 3 below.

**Table 3. Work of adhesion**

| Tablet | Work of adhesion [µJ] |
|---|---|
| Ex. 1 (wet-granulation, furazidin 50 mg) | 0.74 |
| Ex. 2 (direct compression, furazidin 50 mg) | 0.41 |

It can be seen that mucoadhesion of the tablets containing mucoadhesive polymer prepared by wet granulation is much higher than for tablets of the same composition prepared by direct compression. High level of binding and adherence to the mucin, glycoprotein that is the main component of mucosal surfaces, enables to obtain high time of residence at the site of absorption of the drug and thus improve its bioavailability. This is especially important in view of very low solubility of furazidin in water.

For comparative purposes, work of adhesion to the mucin disc in vitro was measured also for furazidin commercial tablets for oral administration. Tested products and work of adhesion are presented in Table 4 below.

**Table 4. Mucoadhesion of commercial tablets for oral administration**

| Product | uroFuragin | uroFuragin Max | Dafurag max |
|---|---|---|---|
| Furazidin strength [mg]. | 50 | 100 | 100 |
| Furazidin concentration in the tablet, % NV/vV | 50% | 33.3% | 24.5% |
| Mass of the tablet [mg] | 100 | 300 | 407,5 |
| Excipients | Corn starch, sucrose, colloidal silica, stearic acid | Lactose monohydrate, silicified microcrystalline cellulose, colloidal silica, magnesium stearate | Mannitol, lactose, colloidal silica, sodium carboxymethylcellulose, magnesium stearate, talc |
| Work of adhesion [µJ] | 0.30 | 0.33 | 0.18 |

It can be seen that commercial tablets designed for oral administration are not suitable for vaginal administration because of low mucoadhesion.

### B. Disintegration time

Disintegration time of the tablets was measured in accordance with European Pharmacopoeia 11.0 test for vaginal tablets 2.9.2. (Disintegration test for rectal and oral dosage forms), disintegration criterium e)).

Disintegration time of the tablets of the invention (Ex. 1) was found to be 3:30 [min:sec].

Tablets of the invention have good disintegration time of the extragranular part. This allows easy and fast spreading and distribution of the furazidin containing granulate matrix throughout the entire vaginal cavity. Therefore, release rate of furazidin from the tablets of the invention is not limited by disintegration of the extragranular phase.

## Claims

1. A pharmaceutical composition of furazidin in the form of a vaginal mucoadhesive tablet, said tablet comprising or consisting of:
a) a granulate containing furazidin, a pharmaceutically acceptable polymeric mucoadhesive binder, and intragranular pharmaceutically acceptable excipients, said excipients comprising or consisting of one or more intragranular fillers and an intragranular disintegrant; and
b) extragranular pharmaceutically acceptable excipients, said excipients comprising or consisting of one or more extragranular fillers, an extragranular disintegrant, and a lubricant,
and wherein the tablet comprises from 1 to 15% by weight of furazidin with respect to the weight of the tablet.

2. The composition of claim 1, wherein the amount of furazidin is from 2.5 to 10% by weight with respect to the weight of the tablet, especially 2.5% by weight or 10% by weight of the tablet.

3. The composition of any one of claims 1 to 2, the amount of the polymeric mucoadhesive binder is in the range from 2 to 7%, especially 3% by weight with respect to the weight of the tablet.

4. The composition of claim 3, wherein the mucoadhesive binder is povidone, especially povidone K30.

5. The composition of any one of claims 1 to 4, wherein said intragranular and/or extragranular one or more fillers is selected from the group consisting of lactose, microcrystalline cellulose and their mixtures.

6. The composition of any one of claims 1 to 5, wherein both intragranular and extragranular one or more fillers is a mixture of lactose and microcrystalline cellulose.

7. The composition of any one of claims 1 to 6, wherein said one or more intragranular fillers is a mixture of microcrystalline cellulose and lactose, especially lactose monohydrate, said one or more extragranular fillers is a mixture of microcrystalline cellulose and lactose, especially lactose monohydrate, and mucoadhesive polymeric binder is povidone, especially povidone K30.

8. The composition of any one of claims 1 to 7, wherein the intragranular and extragranular disintegrant is selected from the group consisting of starch sodium glycolate, corn starch pregelatinised, such as corn starch pregelatinised 1500, low-substituted hydroxypropylcellulose, cellulose microcrystalline, croscarmellose sodium and crospovidone.

9. The composition of claim 8, wherein the intragranular disintegrant and extragranular disintegrant are the same.

10. The composition of claim 9, wherein the intragranular disintegrant is crospovidone and extragranular disintegrant is crospovidone.

11. The composition of any one of claims 1 to 10, wherein the lubricant is magnesium stearate.

12. The composition of any one of claims 1 to 11, wherein a furazidin dose strength is from 2.5 to 100 mg.

13. The composition of claim 12, wherein the furazidin dose strength is selected from the group consisting of 5 mg, 25 mg, 50 mg and 100 mg, preferably is 5 mg.

14. The composition of any one of the preceding claims, wherein the granulate is prepared by wet-granulation, especially by high-shear granulation.

15. A process for the preparation of a pharmaceutical composition of furazidin in the form of a mucoadhesive tablet for vaginal administration as defined in any one of claims 1 to 13, comprising or consisting of the following steps:
a) granulation of the blend of furazidin and intragranular excipients with an aqueous solution of a polymeric mucoadhesive binder to prepare furazidin granulate;
b) adding extragranular excipients to the furazidin granulate to prepare a blend, and
c) compressing the blend into the tablet.

16. The process of claim 15, wherein the granulation is performed in a high-shear granulator.
